# EUROPEAN PATENT APPLICATION

(11) **EP 4 623 708 A1**
(43) Date of publication of application: **01.10.2025**
(21) Application number: 24166520.7
(22) Date of filing: 26.03.2024
(51) Int. Cl.: A24B 13/00, A24B 15/16, A24B 15/28, A24B 15/10, A61K 9/00, A61K 31/465

(54) **SWEETENER COMPOSITION FOR POUCHED ORAL PRODUCTS**

(71) Applicant: Nicoventures Trading Limited, London WC2R 3LA (GB)
(72) Inventor: Gustafson, Johan Robert, Malmo (SE); Keller, Christopher, North Carolina (US)
(74) Representative: Newcombe, Christopher David

(57) **Abstract**

The disclosure provides compositions configured for oral use, such as pouched products. Example pouched products configured for oral use can include a composition enclosed within a water-permeable pouch, the composition including a particulate filler component; one or more sweeteners; and a releasable component selected from the group consisting of flavoring agents, active ingredients, and combinations thereof; wherein the composition is substantially free of sucralose.

## Description

### TECHNICAL FIELD

The present disclosure relates to an oral product. In particular, the present disclosure relates to products intended for human consumption. The products are configured for oral use and deliver substances such as flavors and/or active ingredients during use.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, botanicals, or cannabidiol. The format of such products can vary and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

The present disclosure generally provides compositions configured for oral use and products comprising such compositions. The compositions generally comprise a filler; one or more sweeteners; and a releasable component. The compositions are substantially free of sucralose. Rather, the compositions include one or more sweeteners as alternatives to sucralose, such as other artificial or natural sweeteners or combinations thereof. In some embodiments, the compositions are formulated to avoid undesirable off-flavors or bitterness often associated with artificial sweeteners. Further, the compositions are advantageous by virtue of not including sucralose, which has been shown to accumulate in the biosphere, particularly in aquatic environments.

Accordingly, in one aspect is provided a pouched composition configured for oral use, comprising a composition enclosed within a water-permeable pouch, the composition comprising a particulate filler component; one or more sweeteners; and a releasable component selected from the group consisting of flavoring agents, active ingredients, and combinations thereof, wherein the composition is substantially free of sucralose.

In some embodiments, the one or more sweeteners comprise at least one artificial, non-nutritive sweetener.

In some embodiments, the at least one artificial, non-nutritive sweetener is selected from the group consisting of saccharin, aspartame, neotame, Advantame, cyclamate, acesulfame K, and combinations thereof.

In some embodiments, the one or more sweeteners comprise a sugar alcohol, steviol or an isosteviol glycoside, thaumatin, D-psicose, rubusoside, rebaudioside B, rebaudioside C, trilobatin, phyllodulcin, brazzein, mogrosides, or combinations thereof.

In some embodiments, the one or more sweeteners comprise a sugar alcohol.

In some embodiments, the one or more sweeteners comprises a combination of xylitol, acesulfame K, and saccharin.

In some embodiments, the composition comprises from about 1 to about 5% by weight of xylitol; from about 0.1 to about 0.75% by weight of acesulfame K; and from about 0.05 to about 0.5% by weight of saccharin.

In some embodiments, the particulate filler component comprises a cellulose material, a starch, or both. In some embodiments, the particulate filler component comprises microcrystalline cellulose.

In some embodiments, the releasable component comprises a flavoring agent.

In some embodiments, the releasable component comprises one or more active ingredients selected from the group consisting of nutraceuticals, botanicals, stimulants, amino acids, vitamins, minerals, cannabinoids, cannabimimetics, terpenes, and combinations thereof.

In some embodiments, the releasable component comprises a nicotine component.

In some embodiments, the composition further comprises one or more salts, one or more organic acids, one or more binding agents, one or more buffers, one or more humectants, or combinations thereof.

In some embodiments, the composition further comprises a sweetness enhancer and/or a bitter modulator.

In some embodiments, the composition further comprises a sweetness enhancer. In some embodiments, the sweetness enhancer is a positive allosteric modulator of the T1R2/T1R3 taste receptor.

In some embodiments, the composition further comprises a bitter modulator. In some embodiments, the bitter modulator is selected from the group consisting of gamma-amino butyric acid (GABA), adenosine monophosphate (AMP), and combinations thereof.

In some embodiments, the pouched product has a water content from about 20 to about 60% by weight, based on the weight of the pouched product.

In some embodiments, the pouched product comprises: about 30 to about 75% by weight of microcrystalline cellulose; about 20 to about 60% by weight of water; about 0.1 to about 10% by weight of a nicotine component, calculated as the free base; about 1 to about 5% by weight of xylitol; about 0.1 to about 0.75% by weight of acesulfame K; and about 0.05 to about 0.5% by weight of saccharin, based on the total weight of the composition.

The disclosure includes, without limitations, the following embodiments.

Embodiment 1: A composition configured for oral use, the composition comprising: a particulate filler component; one or more sweeteners; and a releasable component selected from the group consisting of flavoring agents, active ingredients, and combinations thereof; wherein the composition is substantially free of sucralose.

Embodiment 2: The composition of embodiment 1, wherein the one or more sweeteners comprises at least one artificial, non-nutritive sweetener.

Embodiment 3: The composition of embodiment 1 or 2, wherein the one or more sweeteners comprise a sugar alcohol, steviol or an isosteviol glycoside, thaumatin, D-psicose, rubusoside, rebaudioside B, rebaudioside C, trilobatin, phyllodulcin, brazzein, mogrosides, or combinations thereof.

Embodiment 4: The composition of any one of embodiments 1-3, wherein the one or more sweeteners comprise an artificial sweetener selected from the group consisting of saccharin, aspartame, neotame, Advantame, cyclamate, acesulfame K, and combinations thereof.

Embodiment 5: The composition of any one of embodiments 1-4, wherein the one or more sweeteners comprise a sugar alcohol.

Embodiment 6: The composition of any one of embodiments 1-5, wherein the one or more sweeteners comprises a combination of xylitol, acesulfame K, and saccharin.

Embodiment 7: The composition of any one of embodiments 1-6, comprising from about 1 to about 5% by weight of xylitol; from about 0.1 to about 0.75% by weight of acesulfame K; and from about 0.05 to about 0.5% by weight of saccharin.

Embodiment 8: The composition of any one of embodiments 1-7, wherein the particulate filler component comprises a cellulose material, a starch, or both.

Embodiment 9: The composition of any one of embodiments 1-8, wherein the particulate filler component comprises microcrystalline cellulose.

Embodiment 10: The composition of any one of embodiments 1-9, wherein the releasable component comprises a flavoring agent.

Embodiment 11: The composition of any one of embodiments 1-9, wherein the releasable component comprises one or more active ingredients selected from the group consisting of nutraceuticals, botanicals, stimulants, amino acids, vitamins, minerals, cannabinoids, cannabimimetics, terpenes, and combinations thereof.

Embodiment 12: The composition of any one of embodiments 1-9, wherein the releasable component comprises a nicotine component.

Embodiment 13: The composition of any one of embodiments 1-12, further comprising one or more salts, one or more organic acids, one or more binding agents, one or more buffers, one or more humectants, or combinations thereof.

Embodiment 14: The composition of any one of embodiments 1-13, further comprising a sweetness enhancer and/or a bitter modulator.

Embodiment 15: The composition of embodiment 14, wherein the sweetness enhancer is a positive allosteric modulator of the T1R2/T1R3 taste receptor, and optionally when the sweetness enhancer is FEMA 4774.

Embodiment 16: The composition of embodiment 14, wherein the bitter modulator is selected from the group consisting of gamma-amino butyric acid (GABA), adenosine monophosphate (AMP), and combinations thereof.

Embodiment 17: The composition of any one of embodiments 1-16, comprising: about 30 to about 75% by weight of microcrystalline cellulose; about 20 to about 60% by weight of water; about 0.1 to about 10% by weight of a nicotine component, calculated as the free base; about 1 to about 5% by weight of xylitol; about 0.1 to about 0.75% by weight of acesulfame K; and about 0.05 to about 0.5% by weight of saccharin, based on the total weight of the composition.

Embodiment 18: The composition of any one of embodiments 1-17, enclosed in a pouch to form a pouched product, the composition optionally being in a free-flowing, particulate form.

Embodiment 19: The pouched product of embodiment 18, having a water content from about 20 to about 60% by weight, based on the total weight of the pouched product.

Embodiment 20: The composition of any one of embodiments 1-17, in the form of a free-flowing particulate, a tablet, a chew, a melt, a pastille, a lozenge, or a gum.

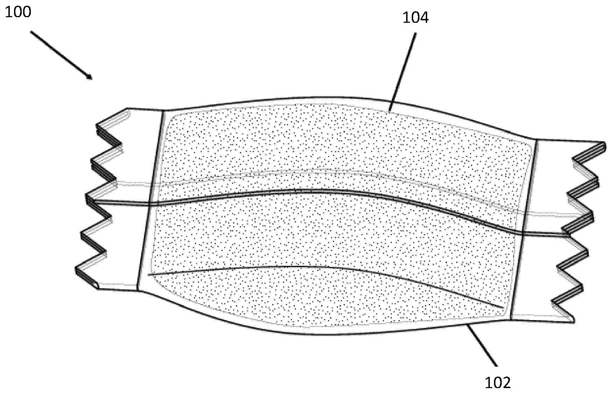

These and other features, aspects, and advantages of the disclosure will be apparent from a reading of the following detailed description together with the accompanying drawing, which is briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

### BRIEF DESCRIPTION OF THE DRAWING

Having thus described aspects of the disclosure in the foregoing general terms, reference will now be made to the accompanying drawing, which is not necessarily drawn to scale. The drawing is exemplary only and should not be construed as limiting the disclosure.

The Figure is a cross-sectional view of a pouched product embodiment, taken across the width of the product, showing an outer pouch filled with a composition of the present disclosure.

### DETAILED DESCRIPTION

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

As used in this specification and the claims, the singular forms "a," "an," and "the" include plural referents unless the context clearly dictates otherwise.

The term "about" used throughout this specification is used to describe and account for small fluctuations. For example, the term "about" can refer to less than or equal to ±10%, such as less than or equal to ±5%, less than or equal to ±2%, less than or equal to ±1%, less than or equal to ±0.5%, less than or equal to ±0.2%, less than or equal to ±0.1% or less than or equal to ±0.05%. All numeric values herein are modified by the term "about," whether or not explicitly indicated. A value modified by the term "about" of course includes the specific value. For instance, "about 5.0" must include 5.0.

Reference to "dry weight percent" or "dry weight basis" refers to weight on the basis of dry ingredients (i.e., all ingredients except water). Reference to "wet weight" refers to the weight of the composition including water. Unless otherwise indicated, reference to "weight percent" of a composition reflects the total wet weight of the composition (i.e., including water).

The present disclosure provides compositions configured for oral use, products comprising such compositions, and methods of preparing the compositions. The compositions generally comprise a particulate filler component, a releasable component such as an active ingredient or flavorant, and one or more sweeteners in the absence of sucralose. Sucralose is a widely used sugar substitute due to its intense sweetness but is increasingly viewed as an environmental pollutant. Sucralose is typically excreted from the body unchanged and likewise passes through municipal wastewater treatment systems without significant degradation. Accordingly, there is a desire to remove sucralose from oral products while maintaining sweetness and other positive sensorial attributes. Each of the compositions, products, methods, and components of each thereof are further described herein below.

### Sweeteners

The composition comprises one or more sweeteners. The sweeteners can be any sweetener or combination of sweeteners, in natural or artificial form, or a combination of natural and artificial sweeteners; however, the composition as disclosed herein is substantially free of sucralose. Sucralose is the generic name for trichlorogalatosucrose, sold under the brand name Splenda^{®}. Sucralose is about 600 times sweeter than sugar but is not digested in the body and is accordingly non-caloric. As noted above, it has recently been reported that sucralose can withstand microbial degradation and is accumulating in the environment. For example, sucralose has been found in wastewater, estuaries, rivers, and the Gulf Stream. See, e.g., Subedi et al., Environ Sci Technol. 2014 Dec 2;48(23): 13668-74; and Naik et al., J Environ Public Health. 2021 Apr 14:2021:6624569. Accordingly, it is desirable to provide compositions substantially free of sucralose at least to reduce environmental accumulation of sucralose.

As used herein, "substantially free" of sucralose means that no sucralose has been added beyond minor amounts that may be present in components of the composition such as flavorings. In some embodiments, the composition includes less than about 0.1% by weight of sucralose, such as less than about 0.05%, less than about 0.01%, less than about 0.001%, less than about 0.0001%, or even 0% by weight of sucralose, based on the total weight of the composition.

To provide the desirable sweetness, compositions of the disclosure include one or more non-sucralose sweeteners. Sweeteners are often classified based on nutritional value, relative sweetness, and source. Examples of suitable sweeteners, which may be nutritive or non-nutritive, include natural sweeteners, artificial sweeteners, and sugar alcohols. Relative sweetness can be experimentally determined in controlled studies comparing the sensory perception of an aqueous solution of various sweeteners to an aqueous solution of sucrose at the same concentration. There are publicly available resources with data regarding the relative sweetness of various sweeteners. See, e.g., Karl F. Tiefenbacher, Wafer and Waffle, Chapter 3, 2017.

As used herein, the term "non-nutritive" refers to a sweetener having no or very low caloric content (e.g., less than 5 calories per gram), and such sweeteners are typically characterized by a higher level of sweetness intensity as compared to many nutritive sweeteners. The low- or non-caloric content of a sweetener may be due to a very high sweetness index such that to provide the desired sweetness to a composition, it is only required in amounts which are insignificant in caloric content, or due to the inability of the body to metabolize the sweetener. Non-nutritive sweeteners may be naturally occurring, such as those obtained from plants, or may be artificial.

In some embodiments, the composition comprises a natural sweetener, such as a sugar, or a glycoside, protein, or other naturally occurring compound which provides a sensation of sweetness in the oral cavity when consumed. In some embodiments, the natural sweetener comprises fructose, sucrose, glucose, maltose, mannose, galactose, lactose, stevia, or a combination thereof. In some embodiments, the natural sweetener comprises steviol, an isosteviol glycoside such as stevioside, dulcoside A, rebaudioside A, rebaudioside B, rebaudioside C, rebaudioside D, rebaudioside M, a mogroside, trilobatin, thaumatin, D-psicose, rubusoside, phyllodulcin, brazzein, or a combination thereof.

When present, the amount of natural sweetener present in the composition may vary. The sweetness of any individual natural sweetener may vary, and may be significantly greater than, approximately the same as, or significantly less than the sweetness of the same concentration of sucrose. In some embodiments, the composition comprises one or more natural sweeteners in an amount from about 0.01 to about 10% by weight, based on the total weight of the composition.

In some embodiments, the composition comprises an artificial sweetener. Artificial sweeteners are typically non-nutritive and provide a sweetness greater than sucrose. Artificial sweeteners may be derived through manufacturing of plant extracts or processed by chemical synthesis. Artificial sweeteners include but are not limited to isomaltulose, maltodextrin, saccharin, aspartame, acesulfame K, and neotame. When present, the amount of artificial sweetener present in the composition may vary. In some embodiments, the composition comprises one or more artificial sweeteners in an amount from about 0.05 to about 1% by weight, based on the total weight of the composition, such as from about 0.05, about 0.1, about 0.2, about 0.3, about 0.4, about 0.5, or about 0.6, to about 0.7, about 0.8, about 0.9, or about 1.0%. In some embodiments, the composition comprises an artificial sweetener selected from the group consisting of saccharin, aspartame, neotame, Advantame, cyclamate, acesulfame K, and combinations thereof. In some embodiments, the composition comprises saccharin, acesulfame K, or a combination thereof. In some embodiments, the composition comprises from about 0.1 to about 0.75% by weight of acesulfame K; and from about 0.05 to about 0.5% by weight of saccharin.

In some embodiments, the composition comprises a sugar alcohol as a sweetener. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the composition comprises a sugar alcohol selected from erythritol, isomalt, maltitol, mannitol, xylitol, sorbitol, and combinations thereof. Sugar alcohols have a relative sweetness below sucrose, and as a result, are often used in higher amounts as compared to many artificial sweeteners. In some embodiments, the composition comprises from about 0.1 to about 10%, from about 1 to about 5%, or from about 1 to about 3% by weight of a sugar alcohol. In some embodiments, the sugar alcohol is xylitol. In some embodiments, the composition comprises from about 1 to about 3% xylitol by weight, based on the total weight of the composition.

Unlike sucrose, most alternative sweeteners, and particularly artificial sweeteners, exhibit undesirable off-tastes as concentration increases, shifting from pleasant (sweet) towards unpleasant (bitter/metallic). See, e.g., Riera et al., American Journal of Physiology-Regulatory, Integrative and Comparative Physiology, Vol. 293, No. 2, 2007. Therefore, selection of sweeteners and combinations of sweeteners in the composition is adapted to provide the desired level of sweetness without significant unpleasant taste sensation. Accordingly, the specific sweeteners, and the total amount of sweetener as well as the quantity of any individual sweetener in the composition may vary. In some embodiments, a combination of different sweeteners is utilized to provide the desired sweetness profile.

In some embodiments, the composition comprises a combination of a sugar alcohol and two or more artificial sweeteners. In some embodiments, the composition comprises a sugar alcohol selected from erythritol, isomalt, maltitol, mannitol, xylitol, sorbitol, and combinations thereof, and a sweetener selected form the group consisting of saccharin, aspartame, neotame, Advantame, cyclamate, acesulfame K, and combinations of two or more thereof.

In some embodiments, the composition comprises a combination of xylitol, acesulfame K, and saccharin as the sweetener. In some embodiments, the composition comprises from about 1 to about 5% by weight of xylitol; from about 0.1 to about 0.75% by weight of acesulfame K; and from about 0.05 to about 0.5% by weight of saccharin. Surprisingly, according to the present disclosure, it has been found that in some embodiments, this combination of sweeteners and the relative amounts thereof provides the desired product sweetness while minimizing bitterness or aftertaste.

In some embodiments, the composition may include a taste modifier, such as a sweetness enhancer or bitter modulator (i.e., blocker) as described further herein below. It is noted that, in the presence of a sweetness enhancer or bitter modulator, the quantity of sweetener(s) present in the composition may be reduced or increased, respectively, relative to the amounts utilized in the absence of such enhancer/modulator. Accordingly, the amount of total sweetener and any individual sweetener may vary from the quantities recited above in the presence of such modifier.

### Taste Modifiers

In order to improve the organoleptic properties of a composition as disclosed herein, the composition may include one or more taste modifiers, which may also be referred to as taste enhancers. Such modifiers can serve to enhance sweetness or suppress the bitterness or "off notes" of an oral composition comprising certain alternative sweeteners such as artificial sweeteners (e.g., saccharin, acesulfame, and the like).

In some embodiments, the composition comprises a taste modifier configured to suppress a bitter taste, enhance a sweet taste, or a combination thereof. In some embodiments, the composition comprises a component (e.g., an active ingredient or sweetener) having a bitter taste, and further comprises a taste enhancer which masks or blocks the perception of the bitter taste, otherwise referred to as a bitter modulator.

In some embodiments, the bitter modulator is a substance which targets receptors in the user's mouth to mask a bitter taste of another component (e.g., of a sweetener or an active ingredient). In some embodiments, the bitter modulator is capsaicin. In some embodiments, the bitter modulator is the amino acid gamma-amino butyric acid (GABA). Studies in mice suggest that GABA may serve function(s) in taste buds in addition to synaptic inhibition. See, e.g., Dvoryanchikov et al., J Neurosci. 2011 Apr 13;31(15):5782-91. Without wishing to be bound by theory, GABA may suppress the perception of certain tastes, such as bitterness. In some embodiments, the bitter modulator is adenosine monophosphate (AMP). AMP is a naturally occurring nucleotide substance which can block bitter food flavors or enhance sweetness. It does not directly alter the bitter flavor but may alter human perception of "bitter" by blocking the associated receptor. In some embodiments, the taste modifier blocks the bitterness associated with certain artificial sweeteners such as cyclamate or saccharine. Accordingly, in some embodiments, the bitter modulator modulates a Taste 2 Receptor (TAS2R) such as TAS2R31 or TAS2R43.

In some embodiments, the taste modifier is a substance which targets receptors in the user's mouth to enhance sweetness (i.e., a sweetness enhancer) of a component of the composition, such as an artificial sweetener. In particular, sweetness enhancers are often characterized as positive allosteric modulators (PAMs) of the T1R (e.g., T1R2/T1R3) taste receptors. See, e.g., Zhang et al., PNAS, Vol. 107, No. 10, 2010. In some embodiments, the sweetness enhancer is a positive allosteric modulator of the T1R2/T1R3 taste receptor. In some embodiments, the sweetness enhancer is FEMA 4774 (CAS: 1359963-68-0; 4-Amino-5-[2,2-dimethyl-3-[(1-methylethyl)amino]-3-oxopropoxy]-2-methyl-3-quinolinecarboxylic acid), which enhances the sweetness of sucrose and sucralose.

When present, a representative amount of taste modifier is about 0.01% by weight or more, about 0.1% by weight or more, or about 1.0% by weight or more, but will typically make up less than about 10% by weight of the total weight of the composition, (e.g., from about 0.01%, about 0.05%, about 0.1%, or about 0.5%, to about 1%, about 5%, or about 10% by weight based on the total weight of the composition).

### Particulate Filler Component

Compositions as described herein typically comprise a particulate filler component. As used herein, the term "particulate" refers to a material in the form of a plurality of individual particles. In various embodiments, the particles of a particulate material (e.g., a particulate filler) can be described as substantially spherical or granular. Materials in particulate form (e.g., a particulate filler) have a particle size. The particle size of a particulate material may be measured by sieve analysis. As the skilled person will readily appreciate, sieve analysis (otherwise known as a gradation test) is a method used to measure the particle size distribution of a particulate material.

Particulate fillers may fulfill multiple functions, such as enhancing certain organoleptic properties such as texture and mouthfeel, enhancing cohesiveness or compressibility of the product, and the like. Generally, the filler is a porous particulate material and often is cellulose-based. For example, suitable fillers are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX^{®} brand filler available from International Fiber Corporation), bran fiber, and mixtures thereof. Non-limiting examples of derivatives of non-tobacco plant material include starches (e.g., from potato, wheat, rice, corn), natural cellulose, and modified cellulosic materials. Additional examples of potential fillers include maltodextrin, dextrose, calcium carbonate, calcium phosphate, lactose, mannitol, xylitol, and sorbitol. Combinations of fillers can also be used.

In some embodiments, the particulate filler comprises a starch, a cellulose material, or both. "Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the composition based on the ability of the starch material to impart a specific organoleptic property to composition. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications and are considered to be "modified" starches. Other starches are obtained and subsequently modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), crosslinking, enzyme treatment, acetylation, hydroxypropylation, and/or partial hydrolysis. Other starches are modified by heat treatments, such as pregelatinization, dextrinization, and/or coldwater swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, starch sodium octenyl succinate.

In some embodiments, the filler comprises or is an inorganic material. Examples of potential inorganic fillers include calcium carbonate, calcium phosphate, and bioceramic materials (e.g., porous hydroxyapatite).

In some embodiments, the particulate filler comprises a cellulose material. One non-limiting example of a suitable cellulose material for use in the products described herein is microcrystalline cellulose ("MCC"). The mcc may be synthetic or semi-synthetic, or it may be obtained entirely from natural celluloses. The mcc may be selected from the group consisting of AVICEL^{®} grades PH-100, PH-102, PH-103, PH-105, PH-112, PH-113, PH-200, PH-300, PH-302, VIVACEL^{®} grades 101, 102, 12, 20 and EMOCEL^{®} grades 50M and 90M, and the like, and mixtures thereof. In some embodiments, the particulate filler component comprises MCC. In some embodiments, the filler component is MCC.

In some embodiments, a particulate filler can be characterized as substantially spherical, such as cellulose spheres. By "substantially spherical" is meant at least a portion of the particulate filler component is in the shape of a sphere and/or is "sphere-like' in shape. As such, "substantially spherical" encompasses slightly elongated (e.g., oval) shapes, slightly flattened shapes, and the like. Substantially spherical particulate filler components are intended to be distinguished from conventional particulate filler components (e.g., commercially available "fillers" or "particulate fillers" that are not explicitly designed as "spherical"). Such conventional particulate filler components typically substantially comprise particles with rather irregular shapes. In some cases, at least some (e.g., including a majority) of such conventional particulate filler components comprise one or more edges (e.g., jagged edges) that are typically not observable on substantially spherical particulate filler components as employed in the context of the present disclosure. Further, the uniformity of the particle shapes and sizes of a substantially spherical filler component is generally much greater than that of a conventional particulate filler component.

In some embodiments, the substantially spherical filler component comprises MCC. In some embodiments, the substantially spherical filler component comprises solid (although porous) MCC spheres. In some embodiments, the substantially spherical filler component comprises hollow MCC spheres. In some embodiments, the center/core of such hollow MCC spheres may be unfilled; in other embodiments, the center/core of such hollow MCC spheres may be filled with one or more additional components (e.g., flavorants, fillers, active ingredients, etc.). Examples of suitable MCC spheres include, but are not limited to, Vivapur^{®} MCC spheres from JRS Pharma, available, e.g., with particle sizes of 100-200 µm (Vivapur^{®} 100), 200-355 µm (Vivapur^{®} 200), 355-500 µm (Vivapur^{®} 350), 500-710 µm (Vivapur^{®} 500), 710-1000 µm (Vivapur^{®}700), and 1000-1400 µm (Vivapur^{®} 1000). Further examples of suitable MCC spheres include, but are not limited to, Celphere^{™} MCC spheres from Asahi Kasei Corporation, available, e.g., with particle sizes of 75-212 µm (Celphere^{™} SCP-100), 106-212 µm (Celphere^{™} CP-102), 150-300 µm (Celphere^{™} CP-203), 300-500 µm (Celphere^{™} CP-305), and 500-710 µm (Celphere^{™} CP-507).

The amount of filler component on a wet weight basis can vary but is typically up to about 75 percent of the total composition by weight. A typical range of filler component within the composition can be from about 10 to about 75 percent by total weight of the composition, for example, from about 10, about 15, about 20, about 25, or about 30, to about 35, about 40, about 45, about 50, about 55, about 60, about 65, about 70, or about 75 weight percent (e.g., about 15 to about 60 weight percent, or about 25 to about 45 weight percent) on a wet weight basis. In some embodiments, the amount of particulate filler component is at least about 10 percent by weight, such as at least about 20 percent, or at least about 25 percent, or at least about 30 percent, or at least about 35 percent, or at least about 40 percent, based on the total weight of the composition. In some embodiments, the particulate filler is MCC, and is present in an amount from about 10 to about 70% by weight, from about 30 to about 65%, or from about 40 to about 60%, based on the total weight of the composition.

### Releasable Component

The compositions as disclosed herein comprise a releasable component. The term "releasable component" as used herein refers to any material in the composition that is releasable therefrom when in contact with the oral cavity of a consumer. The releasable component, in some embodiments, may be absorbed, adsorbed, or otherwise entrained within the composition or a portion thereof. A wide of variety of releasable components may be utilized. In some embodiments, a plurality of releasable components may be used. Suitable releasable agents include, but are not limited to, flavoring agents, active ingredients, and combinations thereof.

### Flavoring Agent

In some embodiments, the composition comprises as a releasable component one or more flavoring agents. As used herein, a "flavoring agent" or "flavorant" is any flavorful or aromatic substance capable of altering the sensory characteristics associated with the composition. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy. Specific types of flavors include, but are not limited to, vanilla, coffee, chocolate/cocoa, cream, mint, spearmint, menthol, peppermint, wintergreen, eucalyptus, lavender, cardamon, nutmeg, cinnamon, clove, cascarilla, sandalwood, honey, jasmine, ginger, anise, sage, licorice, lemon, orange, apple, peach, lime, cherry, strawberry, and any combinations thereof. See also, Leffingwell et al., Tobacco Flavoring for Smoking Products, R. J. Reynolds Tobacco Company (1972), which is incorporated herein by reference. Flavorings also may include components that are considered moistening, cooling or smoothening agents, such as eucalyptus. These flavors may be provided neat (i.e., alone) or in a composite, and may be employed as concentrates or flavor packages (e.g., spearmint and menthol, orange and cinnamon; lime, tropical, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al., each of which is incorporated herein by reference. In some instances, the flavoring agent may be provided in a spray-dried form or a liquid form.

The flavoring agent generally comprises at least one volatile flavor component. As used herein, "volatile" refers to a chemical substance that forms a vapor readily at ambient temperatures (i.e., a chemical substance that has a high vapor pressure at a given temperature relative to a nonvolatile substance). Typically, a volatile flavor component has a molecular weight below about 400 and will often include functional groups such as alcohols, aldehydes, aromatic hydrocarbons, ketones, esters, terpenes, terpenoids, and combinations thereof. Non-limiting examples of aldehydes include vanillin, ethyl vanillin, p-anisaldehyde, hexanal, furfural, isovaleraldehyde, cuminaldehyde, benzaldehyde, and citronellal. Non-limiting examples of ketones include 1-hydroxy-2-propanone and 2-hydroxy-3-methyl-2-cyclopentenone-1-one. Non-limiting examples of terpenes include sabinene, limonene, gamma-terpinene, beta-farnesene, nerolidol, thujone, myrcene, geraniol, nerol, citronellol, linalool, and eucalyptol. Non-limiting examples of esters include allyl hexanoate, ethyl heptanoate, ethyl hexanoate, isoamyl acetate, and 3-methylbutyl acetate.

The amount of flavoring agent utilized in the composition can vary, but is typically up to about 10 weight percent, and some embodiments are characterized by a flavoring agent content of at least about 0.5 weight percent, such as about 0.5 to about 10 weight percent, about 1 to about 6 weight percent, or about 2 to about 5 weight percent based on the total weight of the composition.

### Active Ingredient

In some embodiments, the composition comprises an active ingredient as the releasable component. As used herein, an "active ingredient" refers to one or more substances belonging to any of the following categories: API (active pharmaceutical substances), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the active ingredient may be of the type generally referred to as dietary supplements, nutraceuticals, "phytochemicals" or "functional foods". These types of additives are sometimes defined in the art as encompassing substances typically available from naturally occurring sources (e.g., botanical materials) that provide one or more advantageous biological effects (e.g., health promotion, disease prevention, or other medicinal properties), but are not classified or regulated as drugs.

Non-limiting examples of active ingredients include those falling in the categories of botanical ingredients, stimulants, amino acids, nicotine components, and/or pharmaceutical, nutraceutical, and medicinal ingredients (e.g., vitamins, such as B6, B12, and C, and/or cannabinoids, such as tetrahydrocannabinol (THC) and cannabidiol (CBD)). Each of these categories is further described herein below. The particular choice of active ingredients will vary depending upon the desired flavor, texture, and desired characteristics of the particular oral product.

The particular percentages of active ingredients present will vary depending upon the desired characteristics of the particular composition. Typically, an active ingredient or combination thereof is present in a total concentration of at least about 0.001% by weight of the composition, such as in a range from about 0.001% to about 20%. In some embodiments, the active ingredient or combination of active ingredients is present in a concentration from about 0.1% w/w to about 10% by weight, such as, e.g., from about from about 0.5% w/w to about 10%, from about 1% to about 10%, from about 1% to about 5% by weight, based on the total weight of the composition. In some embodiments, the active ingredient or combination of active ingredients is present in a concentration of from about 0.001%, about 0.01%, about 0.1% , or about 1%, up to about 20% by weight, such as, e.g., from about from about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight, based on the total weight of the composition. Further suitable ranges for specific active ingredients are provided herein below.

### Nicotine Component

In some embodiments, the active ingredient comprises a nicotine component. By "nicotine component" is meant any suitable form of nicotine (e.g., free base or salt) for providing oral absorption of at least a portion of the nicotine present. The source of the nicotine may vary and may be natural or synthetic. Nicotine may be tobacco-derived (e.g., a tobacco extract) or non-tobacco derived (e.g., synthetic or otherwise obtained). Most preferably, the nicotine is naturally occurring and obtained as an extract from a *Nicotiana* species (e.g., tobacco). The nicotine can have the enantiomeric form *S*-(-)-nicotine, *R*-(+)-nicotine, or a mixture of *S*-(-)-nicotine and *R*-(+)-nicotine. Most preferably, the nicotine is in the form of *S*-(-)-nicotine (e.g., in a form that is virtually all S-(-)-nicotine) or a racemic mixture composed primarily or predominantly of *S*-(-)-nicotine (e.g., a mixture composed of about 95 weight parts *S-*(-)-nicotine and about 5 weight parts *R*-(+)-nicotine). Most preferably, the nicotine is employed in virtually pure form or in an essentially pure form. Highly preferred nicotine that is employed has a purity of greater than about 95 percent, more preferably greater than about 98 percent, and most preferably greater than about 99 percent, on a weight basis.

Typically, the nicotine component is selected from the group consisting of nicotine free base and a nicotine salt. In some embodiments, the nicotine component is nicotine in its free base form, which easily can be adsorbed in for example, a microcrystalline cellulose material to form a microcrystalline cellulose-nicotine carrier complex. See, for example, the discussion of nicotine in free base form in US Pat. Pub. No. 2004/0191322 to Hansson, which is incorporated herein by reference.

In some embodiments, at least a portion of the nicotine component can be employed in the form of a salt. Salts of nicotine can be provided using the types of ingredients and techniques set forth in US Pat. No. 2,033,909 to Cox et al. and Perfetti, Beitrage Tabakforschung Int., 12: 43-54 (1983), which are incorporated herein by reference. Additionally, salts of nicotine are available from sources such as Pfaltz and Bauer, Inc. and K&K Laboratories, Division of ICN Biochemicals, Inc. Typically, the nicotine component is selected from the group consisting of nicotine free base, a nicotine salt such as hydrochloride, dihydrochloride, monotartrate, bitartrate, sulfate, salicylate, and nicotine zinc chloride. In some embodiments, the nicotine component is nicotine bitartrate.

In some embodiments, at least a portion of the nicotine can be in the form of a resin complex of nicotine, where nicotine is bound in an ion-exchange resin, such as nicotine polacrilex, which is nicotine bound to, for example, a polymethacrylic acid, such as Amberlite IRP64, Purolite C115HMR, or Doshion P551. See, for example, US Pat. No. 3,901,248 to Lichtneckert et al., which is incorporated herein by reference. Another example is a nicotine-polyacrylic carbomer complex, such as with Carbopol 974P. In some embodiments, nicotine may be present in the form of a nicotine polyacrylic complex.

In some embodiments, at least a portion of the nicotine component can be ion paired with an organic acid, salt of an organic acid, or combination thereof as described further herein below. Ion pairing is further described in, for example, International Patent Application Publication No. WO 2021050741 to Poole et al., which is incorporated herein by reference.

Typically, the nicotine component (calculated as the free base) when present, is in a concentration of at least about 0.001% by weight of the composition, such as in a range from about 0.001% to about 10%. In some embodiments, the nicotine component is present in a concentration from about 0.1% w/w to about 10% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the composition. In some embodiments, the nicotine component is present in a concentration from about 0.1% w/w to about 3% by weight, such as, e.g., from about 0.1% w/w to about 2.5%, from about 0.1% to about 2.0%, from about 0.1% to about 1.5%, or from about 0.1% to about 1% by weight, calculated as the free base and based on the total weight of the composition. The total amount of nicotine present may be provided by more than one source of nicotine, such as various combinations of any of nicotine free base, nicotine salt, ion paired nicotine, and polymer-bound nicotine (e.g., nicotine polacrilex). In some embodiments, the nicotine component is nicotine benzoate, nicotine polacrilex, or a mixture thereof.

In some embodiments, the product or composition of the disclosure can be characterized as completely free or substantially free of any nicotine component (e.g., any embodiment as disclosed herein may be completely or substantially free of any nicotine component). By "substantially free" is meant that no nicotine has been intentionally added, beyond trace amounts that may be naturally present in e.g., a botanical material. For example, some embodiments can be characterized as having less than 0.001% by weight of nicotine, or less than 0.0001%, or even 0% by weight of nicotine, calculated as the free base.

### Botanical

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any *Nicotiana* species). The botanical materials used in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods."

When present, a botanical is typically at a concentration of from about 0.01% w/w to about 10% by weight, such as, e.g., from about from about 0.01% w/w, about 0.05%, about 0.1%, or about 0.5%, to about 1%, about 2%, about 3%, about 4%, about 5%, about 6%, about 7%, about 8%, about 9%, or about 10%, about 11%, about 12%, about 13%, about 14%, or about 15% by weight, based on the total weight of the composition.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein.

Non-limiting examples of non-tobacco botanical materials include without limitation acai berry (Euterpe oleracea martius), acerola (Malpighia glabra), alfalfa, allspice, Angelica root, anise (e.g., star anise), annatto seed, apple (*Malus domestica*)*,* apricot oil, ashwagandha, *Bacopa monniera,* baobab, basil (*Ocimum basilicum*)*,* bay, bee balm, beet root, bergamot, blackberry (*Morus nigra*)*,* black cohosh, black pepper, black tea, blueberries, boldo (*Peumus boldus*)*,* borage, bugleweed, cacao, calamus root, camu (*Myrcaria dubia*)*,* cannabis/hemp, caraway seed, cardamom, cassis, catnip, catuaba, cayenne pepper, *Centella asiatica,* chaga mushroom, Chai-hu, chamomile, cherry, chervil, chive, chlorophyll, chocolate, cilantro, cinnamon (*Cinnamomum cassia*)*,* citron grass (*Cymbopogon citratus*)*,* citrus, clary sage, cloves, coconut (*Cocos nucifera*)*,* coffee, comfrey leaf and root, cordyceps, coriander seed, cranberry, cumin, curcumin, damiana, dandelion, *Dorstenia arifolia, Dorstenia odorata,* Echinacea, elderberry, elderflower, endro (*Anethum graveolens*)*,* evening primrose, eucalyptus, fennel, feverfew, flax, *Galphimia glauca,* garlic, ginger *(Zingiber officinale*)*,* gingko biloba, ginseng, goji berries, goldenseal, grape seed, grapefruit, grapefruit rosé (*Citrus paradisi*)*,* graviola (*Annona muricata*)*,* green tea, guarana, gutu kola, hawthorn, hazel, hemp, hibiscus flower (*Hibiscus sabdariffa*)*,* honeybush, hops, jiaogulan, jambu (*Spilanthes oleraceae*)*,* jasmine (*Jasminum officinale*)*,* juniper berry (*Juniperus communis*)*, Kaempferia parviflora* (Thai ginseng), kava, laurel, lavender, lemon (*Citrus limon*), lemon balm, lemongrass, licorice, lilac, Lion's mane, lutein, maca (*Lepidium meyenii*), mace, marjoram, matcha, milk thistle, mints (menthe), mulberry, *Nardostachys chinensis,* nutmeg, olive, oolong tea, orange (*Citrus sinensis*)*,* oregano, papaya, paprika, pennyroyal, peppermint (*Mentha piperita*)*,* pimento, potato peel, primrose, quercetin, quince, red clover, resveratrol, *Rhizoma gastrodiae, Rhodiola,* rooibos (red or green), rosehip (*Rosa canina*)*,* rosemary, saffron, sage, Saint John's Wort, sandalwood, salvia (*Salvia officinalis*)*,* savory, saw palmetto, *Sceletium tortuosum,* Schisandra, silybum marianum, Skullcap, spearmint, Spikenard, spirulina, slippery elm bark, sorghum bran hi-tannin, sorghum grain hi-tannin, spearmint (*Mentha spicata*)*,* spirulina, star anise, sumac bran, tarragon, thyme, tisanes, turmeric, *Turnera aphrodisiaca,* uva ursi, valerian, vanilla, *Viola odorata,* wild yam root, wintergreen, withania somnifera, yacon root, yellow dock, yerba mate, and yerba santa.

### Stimulants

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis. In some embodiments, the active ingredient comprises caffeine. In some embodiments, the active ingredient is caffeine. In some embodiments, the caffeine is present in an encapsulated form. On example of an encapsulated caffeine is Vitashure^{®}, available from Balchem Corp., 52 Sunrise Park Road, New Hampton, NY, 10958.

When present, a stimulant or combination of stimulants (e.g., caffeine, theacrine, and combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition.

### Amino Acids

In some embodiments, the active ingredient comprises an amino acid. As used herein, the term "amino acid" refers to an organic compound that contains amine (-NH₂) and carboxyl (-COOH) or sulfonic acid (SOsH) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein or is not directly produced by cellular machinery (e.g., is the product of post-translational modification). Non-limiting examples of non-proteinogenic amino acids include gamma-aminobutyric acid (GABA), taurine (2-aminoethanesulfonic acid), theanine (L-γ-glutamylethylamide), hydroxyproline, and beta-alanine.

When present, an amino acid or combination of amino acids (e.g., taurine, theanine, and combinations thereof) is typically at a concentration of from about 0.1% w/w to about 15% by weight, such as, e.g., from about from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition.

### Vitamins and Minerals

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones). In some embodiments, the active ingredient comprises vitamin C. In some embodiments, the active ingredient is a combination of vitamin C, caffeine, and taurine. In some embodiments, the active ingredient comprises one or more of vitamin B6 and B12. In some embodiments, the active ingredient comprises theanine and one or more of vitamin B6 and B12.

When present, a vitamin or combination of vitamins (e.g., vitamin B6, vitamin B12, vitamin E, vitamin C, or a combination thereof) is typically at a concentration of from about 0.01% w/w to about 1% by weight, such as, e.g., from about 0.01%, about 0.02%, about 0.03%, about 0.04%, about 0.05%, about 0.06%, about 0.07%, about 0.08%, about 0.09%, or about 0.1% w/w, to about 0.2%, about 0.3%, about 0.4%, about 0.5% about 0.6%, about 0.7%, about 0.8%, about 0.9%, or about 1% by weight, based on the total weight of the composition.

In some embodiments, the active ingredient comprises vitamin A. In some embodiments, the vitamin A is encapsulated. In some embodiments, the vitamin is vitamin B6, vitamin B12, vitamin E, vitamin C, or a combination thereof.

In some embodiments, the active ingredient comprises a mineral. As used herein, the term "mineral" refers to an inorganic molecule (or related set of molecules) that is an essential micronutrient needed for the proper functioning of various systems in a mammal. Non-limiting examples of minerals include iron, zinc, copper, selenium, chromium, cobalt, manganese, calcium, phosphorus, sulfur, magnesium, and the like. In some embodiments, the active ingredient comprises iron. Suitable sources of iron include, but are not limited to, ferrous salts such as ferrous sulfate and ferrous gluconate. In some embodiments, the iron is encapsulated.

### Cannabinoids

In some embodiments, the active ingredient comprises one or more cannabinoids. As used herein, the term "cannabinoid" refers to a class of diverse natural or synthetic chemical compounds that acts on cannabinoid receptors (i.e., CB1 and CB2) in cells that alter neurotransmitter release in the brain. Cannabinoids are cyclic molecules exhibiting particular properties such as the ability to easily cross the blood-brain barrier. Cannabinoids may be naturally occurring (Phytocannabinoids) from plants such as cannabis, (endocannabinoids) from animals, or artificially manufactured (synthetic cannabinoids). Cannabis species express at least 85 different phytocannabinoids, and these may be divided into subclasses, including cannabigerols, cannabichromenes, cannabidiols, tetrahydrocannabinols, cannabinols and cannabinodiols, and other cannabinoids, such as cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabmolic acid (THCA), and tetrahydrocannabivarinic acid (THCV A).

In some embodiments, the cannabinoid is selected from the group consisting of cannabigerol (CBG), cannabichromene (CBC), cannabidiol (CBD), tetrahydrocannabinol (THC), cannabinol (CBN) and cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabinolic acid (THCA), tetrahydrocannabivarinic acid (THCV A), and mixtures thereof. In some embodiments, the cannabinoid comprises at least tetrahydrocannabinol (THC). In some embodiments, the cannabinoid is tetrahydrocannabinol (THC). In some embodiments, the cannabinoid comprises at least cannabidiol (CBD). In some embodiments, the cannabinoid is cannabidiol (CBD). In some embodiments, the CBD is synthetic CBD.

In some embodiments, the cannabinoid (e.g., CBD) is added to the composition in the form of an isolate. An isolate is an extract from a plant, such as cannabis, where the active material of interest (in this case the cannabinoid, such as CBD) is present in a high degree of purity, for example greater than 95%, greater than 96%, greater than 97%, greater than 98%, or around 99% purity.

In some embodiments, the cannabinoid is an isolate of CBD in a high degree of purity, and the amount of any other cannabinoid in the composition is no greater than about 1% by weight of the composition, such as no greater than about 0.5% by weight of the composition, such as no greater than about 0.1% by weight of the composition, such as no greater than about 0.01% by weight of the composition.

The choice of cannabinoid and the particular percentages thereof which may be present within the disclosed composition will vary depending upon the desired flavor, texture, and other characteristics of the composition.

In some embodiments, the cannabinoid (such as CBD) is present in the composition in a concentration of at least about 0.001% by weight of the composition, such as in a range from about 0.001% to about 2% by weight of the composition. In some embodiments, the cannabinoid (such as CBD) is present in the composition in a concentration of from about 0.1% to about 1.5% by weight, based on the total weight of the composition. In some embodiments, the cannabinoid (such as CBD) is present in a concentration from about 0.4% to about 1.5% by weight, based on the total weight of the oral composition.

Alternatively, or in addition to the cannabinoid, the active ingredient may include a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids. Such compounds can be used in the same amounts and ratios noted herein for cannabinoids.

### Terpenes

Active ingredients suitable for use in the present disclosure can also be classified as terpenes, many of which are associated with biological effects, such as calming effects. Terpenes are understood to have the general formula of (C₅H₈)ₙ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

In some embodiments, the terpene is a terpene derivable from a phytocannabinoid producing plant, such as a plant from the stain of the cannabis sativa species, such as hemp. Suitable terpenes in this regard include so-called "C10" terpenes, which are those terpenes comprising 10 carbon atoms, and so-called "C15" terpenes, which are those terpenes comprising 15 carbon atoms. In some embodiments, the active ingredient comprises more than one terpene. For example, the active ingredient may comprise one, two, three, four, five, six, seven, eight, nine, ten or more terpenes as defined herein. In some embodiments, the terpene is selected from pinene (alpha and beta), geraniol, linalool, limonene, carvone, eucalyptol, menthone, iso-menthone, piperitone, myrcene, beta-bourbonene, germacrene and mixtures thereof.

### Antioxidants

In some embodiments, the active ingredient comprises one or more antioxidants. As used herein, the term "antioxidant" refers to a substance which prevents or suppresses oxidation by terminating free radical reactions and may delay or prevent some types of cellular damage. Antioxidants may be naturally occurring or synthetic. Naturally occurring antioxidants include those found in foods and botanical materials. Non-limiting examples of antioxidants include certain botanical materials, vitamins, polyphenols, and phenol derivatives.

Examples of botanical materials which are associated with antioxidant characteristics include without limitation acai berry, alfalfa, allspice, annatto seed, apricot oil, basil, bee balm, wild bergamot, black pepper, blueberries, borage seed oil, bugleweed, cacao, calamus root, catnip, catuaba, cayenne pepper, chaga mushroom, chervil, cinnamon, dark chocolate, potato peel, grape seed, ginseng, gingko biloba, Saint John's Wort, saw palmetto, green tea, black tea, black cohosh, cayenne, chamomile, cloves, cocoa powder, cranberry, dandelion, grapefruit, honeybush, echinacea, garlic, evening primrose, feverfew, ginger, goldenseal, hawthorn, hibiscus flower, jiaogulan, kava, lavender, licorice, marjoram, milk thistle, mints (menthe), oolong tea, beet root, orange, oregano, papaya, pennyroyal, peppermint, red clover, rooibos (red or green), rosehip, rosemary, sage, clary sage, savory, spearmint, spirulina, slippery elm bark, sorghum bran hi-tannin, sorghum grain hi-tannin, sumac bran, comfrey leaf and root, goji berries, gutu kola, thyme, turmeric, uva ursi, valerian, wild yam root, wintergreen, yacon root, yellow dock, yerba mate, yerba santa, bacopa monniera, withania somnifera, Lion's mane, and silybum marianum. Such botanical materials may be provided in fresh or dry form, essential oils, or may be in the form of an extracts. The botanical materials (as well as their extracts) often include compounds from various classes known to provide antioxidant effects, such as minerals, vitamins, isoflavones, phytoesterols, allyl sulfides, dithiolthiones, isothiocyanates, indoles, lignans, flavonoids, polyphenols, and carotenoids. Examples of compounds found in botanical extracts or oils include ascorbic acid, peanut endocarb, resveratrol, sulforaphane, beta-carotene, lycopene, lutein, coenzyme Q, carnitine, quercetin, kaempferol, and the like. See, e.g., Santhosh et al., Phytomedicine, 12(2005) 216-220, which is incorporated herein by reference.

Non-limiting examples of other suitable antioxidants include citric acid, Vitamin E or a derivative thereof, a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, butylated hydroxyanisole (BHA), butylated hydroxytoluene (BHT), tertiary butylhydroquinone (TBHQ), and combinations thereof.

When present, an antioxidant is typically at a concentration of from about 0.001% w/w to about 10% by weight, such as, e.g., from about from about 0.001%, about 0.005%, about 0.01% w/w, about 0.05%, about 0.1%, or about 0.5%, to about 1%, about 2%, about 3%, about 4%, about 5%, about 6%, about 7%, about 8%, about 9%, or about 10%, based on the total weight of the composition.

### Pharmaceutical Ingredients

In some embodiments, the active ingredient comprises an active pharmaceutical ingredient (API). The API can be any known agent adapted for therapeutic, prophylactic, or diagnostic use. These can include, for example, synthetic organic compounds, proteins and peptides, polysaccharides and other sugars, lipids, phospholipids, inorganic compounds (e.g., magnesium, selenium, zinc, nitrate), neurotransmitters or precursors thereof (e.g., serotonin, 5-hydroxytryptophan, oxitriptan, acetylcholine, dopamine, melatonin), and nucleic acid sequences, having therapeutic, prophylactic, or diagnostic activity. Non-limiting examples of APIs include analgesics and antipyretics (e.g., acetylsalicylic acid, acetaminophen, 3-(4-isobutylphenyl)propanoic acid), phosphatidylserine, myoinositol, docosahexaenoic acid (DHA, Omega-3), arachidonic acid (AA, Omega-6), S-adenosylmethionine (SAM), beta-hydroxy-beta-methylbutyrate (HMB), citicoline (cytidine-5'-diphosphate-choline), and cotinine.

The amount of API may vary. For example, when present, an API is typically at a concentration of from about 0.001% w/w to about 10% by weight, such as, e.g., from about from about 0.01%, about 0.02%, about 0.03%, about 0.04%, about 0.05%, about 0.06%, about 0.07%, about 0.08%, about 0.09%, about 0.1% w/w, about 0.2%, about 0.3%, about 0.4%, about 0.5% about 0.6%, about 0.7%, about 0.8%, about 0.9%, or about 1%, to about 2%, about 3%, about 4%, about 5%, about 6%, about 7%, about 8%, about 9%, or about 10% by weight, based on the total weight of the composition.

### Organic Acid

In some embodiments, the composition as disclosed herein comprises an organic acid, an alkali metal salt thereof, or both. In some embodiments, at least a portion of the organic acid or salt thereof is in the form of an ion pair with a basic amine-containing active ingredient (e.g., nicotine).

As used herein, the term "organic acid" refers to an organic (i.e., carbon-based) compound that is characterized by acidic properties. Typically, organic acids are relatively weak acids (i.e., they do not dissociate completely in the presence of water), such as carboxylic acids (-CO₂H) or sulfonic acids (-SO₂OH). As used herein, reference to organic acid means an organic acid that is intentionally added. In this regard, an organic acid may be intentionally added as a specific composition ingredient as opposed to merely being inherently present as a component of another composition ingredient (e.g., the small amount of organic acid which may inherently be present in a composition ingredient, such as a tobacco material).

Suitable organic acids will typically have a range of lipophilicities (i.e., a polarity giving an appropriate balance of water and organic solubility). Typically, lipophilicities of suitable organic acids, as indicated by logP, will vary between about 1 and about 12 (more soluble in octanol than in water). In some embodiments, the organic acid has a logP value from about 1 to about 12, e.g., from about 1.0. about 1.5, about 2.0, about 2.5, about 3.0, about 3.5, about 4.0, about 4.5, about 5.0, about 5.5, about 6.0, about 6.5, about 7.0, about 7.5, or about 8.0, to about 8.5, about 9.0, about 9.5, about 10.0, about 10.5, about 11.0, about 11.5, or about 12.0.

Without wishing to be bound by theory, it is believed that moderately lipophilic organic acids (e.g., logP of from about 1.4 to about 4.5) produce ion pairs with nicotine which are of a polarity providing good octanol-water partitioning of the ion pair, and hence partitioning of nicotine, into octanol versus water. As discussed above, such partitioning into octanol is predictive of favorable oral availability.

In some embodiments, the organic acid has a logP value from about 3.0 to about 8.0, about 10.0, or even 12.0. In some embodiments, the presence of certain solvents or solubilizing agents (e.g., inclusion in the composition of glycerin or propylene glycol) may be beneficial in solubilizing organic acids and the corresponding salts or ion pairs thereof with the basic amine for highly lipophilic organic acids (e.g., higher than about 4.5).

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms (C1-C20). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all sp3 carbon atoms), or may be unsaturated (i.e., having at least one site of unsaturation). As used herein, the term "unsaturated" refers to the presence of a carbon-carbon, sp² double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted.

"Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted.

"Heteroaryl" and "heterocycloalkyl" as used herein refer to an aromatic or non-aromatic ring system, respectively, in which one or more ring atoms is a heteroatom, e.g. nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, -OH,-OCH₃, NH₂, -NHCH₃, -N(CH₃)₂, -CN, -NC(=O)CH₃, -C(=O)-, -C(=O)NH₂, and -C(=O)N(CH₃)₂. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and the like.

In some embodiments, the organic acid is an alkyl sulfonic acid. Non-limiting examples of alkyl sulfonic acids include propanesulfonic acid, heptanesulfonic acid, and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid,and p-toluenesulfonic acid.

Further non-limiting examples of organic acids which may be useful in some embodiments include dibenzoyl-L-tartaric acid, 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid. Examples of suitable acids include, but are not limited to, the list of organic acids in **Table 1.**

**Table 1. Non-limiting examples of suitable organic acids**

| **Acid Name** | **log(P)*** |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-(4-Isobutylphenyl)propanoic acid | 3.5 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2*E*,4*E*,6*E*,8*E*,10*E*,12*E*,14*E*,16*E*,18*E*)-4,8,13,17-tetramethylicosa-2,4,6,8,10,12,14,16,18-nonaenedioic acid) | 7.2 |
| bixin ((2E,4E,6E,8E,10E,12E,14E,16Z,18E)-20-methoxy-4,8,13,17-tetramethyl-20-oxoicosa-2,4,6,8,10,12,14,16,18-nonaenoic acid) | 7.5 |

| | |
|---|---|
| *Values obtained from PubChem or calculated | |

The selection of organic acid may further depend on additional properties in addition to consideration of the logP value. For example, an organic acid should be one recognized as safe for human consumption, and which has acceptable flavor, odor, volatility, stability, and the like. Determination of appropriate organic acids is within the purview of one of skill in the art.

In some embodiments, the organic acid is a mono ester of a dicarboxylic acid or a polycarboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in oral compositions as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in oral compositions as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in oral compositions by virtue of providing both ion pairing and serving as a colorant in the composition. In some embodiments, the organic acid is 2*E,*4*E,*6*E,* 8*E,*10*E*,12*E*,14*E*,16*Z*,18*E*)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (*Bixa orellana*) and is the naturally occurring pigment providing the reddish orange color to annatto. Bixin is soluble in fats and alcohols but insoluble in water, and is chemically unstable when isolated, converting via isomerization into the double bond isomer, trans-bixin (β-bixin), having the structure:

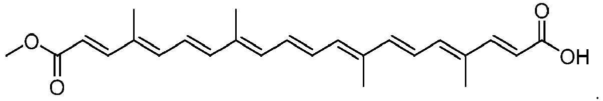

In some embodiments, the organic acid is (2*E*,4*E*,6*E*,8*E*,10*E*,12*E*,14*E*,16*E*,18*E*)-4,8,13,17-tetramethylicosa-2,4,6,8,10,12,14,16,18-nonaenedioic acid (norbixin), a water-soluble hydrolysis product of bixin having the structure:

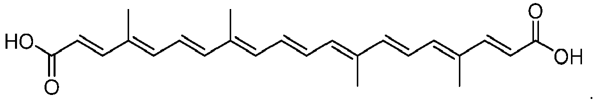

The selection of organic acid may further depend on additional properties in addition to or without consideration to the logP value. For example, an organic acid should be one recognized as safe for human consumption, and which has acceptable flavor, odor, volatility, stability, and the like. Determination of appropriate organic acids is within the purview of one of skill in the art.

In some embodiments, more than one organic acid may be present. For example, the composition may comprise two, or three, or four, or more organic acids. Accordingly, reference herein to "an organic acid" contemplates mixtures of two or more organic acids. The relative amounts of the multiple organic acids may vary. For example, a composition may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids in the composition which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid (e.g., in equimolar or greater quantities relative to nicotine). Without wishing to be bound by theory, it is believed that a combination of different organic acids may provide the desired ion pairing while the concentration of any single organic acid in the composition remains below the threshold which would be found objectionable from a sensory perspective.

For example, in some embodiments, the organic acid may comprise from about 1 to about 5 or more molar equivalents of benzoic acid relative to nicotine, combined with e.g., about 0.2 molar equivalents of octanoic acid or a salt thereof, and 0.2 molar equivalents of decanoic acid or a salt thereof.

In some embodiments, the organic acid is a combination of any two organic acids selected from the group consisting of benzoic acid, a toluic acid, benzenesulfonic acid, toluenesulfonic acid, hexanoic acid, heptanoic acid, decanoic acid, and octanoic acid. In some embodiments, the organic acid is a combination of benzoic acid, octanoic acid, and decanoic acid, or benzoic and octanoic acid. In some embodiments, the composition comprises citric acid in addition to one or more of benzoic acid, a toluic acid, benzenesulfonic acid, toluenesulfonic acid, hexanoic acid, heptanoic acid, decanoic acid, and octanoic acid.

In some embodiments, the oral composition comprises an alkali metal salt of an organic acid. For example, at least a portion of the organic acid may be present in the composition in the form of an alkali metal salt. Suitable alkali metal salts include lithium, sodium, and potassium. In some embodiments, the alkali metal is sodium or potassium. In some embodiments, the alkali metal is sodium. In some embodiments, the composition comprises an organic acid and a sodium salt of the organic acid.

In some embodiments, the oral composition comprises benzoic acid and sodium benzoate, octanoic acid and sodium octanoate, decanoic acid and sodium decanoate, or a combination thereof. In some embodiments, the composition comprises benzoic acid and sodium benzoate. In some embodiments, the composition comprises sodium benzoate.

In some embodiments, the ratio of the organic acid to the sodium salt (or other alkali metal salt) of the organic acid is from about 0.1 to about 10, such as from about 0.1, about 0.25, about 0.3, about 0.5, about 0.75, or about 1, to about 2, about 5, or about 10. For example, in some embodiments, both an organic acid and the sodium salt thereof are added to the other components of the composition, wherein the organic acid is added in excess of the sodium salt, in equimolar quantities with the sodium salt, or as a fraction of the sodium salt. One of skill in the art will recognize that the relative amounts will be determined by the desired pH of the composition, as well as the desired ionic strength. For example, the organic acid may be added in a quantity to provide a desired pH level of the composition, while the alkali metal (e.g., sodium) salt is added in a quantity to provide the desired extent of ion pairing. As one of skill in the art will understand, the quantity of organic acid (i.e., the protonated form) present in the composition, relative to the alkali metal salt or conjugate base form present in the composition, will vary according to the pH of the composition and the pKa of the organic acid, as well as according to the actual relative quantities initially added to the composition.

The amount of organic acid or an alkali metal salt thereof present in the composition, relative to the basic amine containing active ingredient (e.g., nicotine), may vary. Generally, as the concentration of the organic acid (or the conjugate base thereof) increases, the percent of nicotine that is ion paired with the organic acid increases. This typically increases the partitioning of the nicotine, in the form of an ion pair, into octanol versus water as measured by the logP (the log10 of the partitioning coefficient). In some embodiments, the composition comprises from about 0.05, about 0.1, about 1, about 1.5, about 2, or about 5, to about 10, about 15, or about 20 molar equivalents of the organic acid, the alkali metal salt thereof, or the combination thereof, relative to the nicotine component, calculated as free base nicotine.

In some embodiments, the composition comprises from about 2 to about 10, or from about 2 to about 5 molar equivalents of the organic acid, the alkali metal salt thereof, or the combination thereof, to nicotine, on a free-base nicotine basis. In some embodiments, the organic acid, the alkali metal salt thereof, or the combination thereof, is present in a molar ratio with the nicotine from about 2, about 3, about 4, or about 5, to about 6, about 7, about 8, about 9, or about 10. In embodiments wherein more than one organic acid, alkali metal salt thereof, or both, are present, it is to be understood that such molar ratios reflect the totality of the organic acids present.

In some embodiments the organic acid inclusion is sufficient to provide a pH of the composition from about 4.0 to about 9.5, such as from about 4.0 to about 9.0, or from about 4.0 to about 8.5, or from about 4.0 to about 8.0, or from about 4.5 to about 7.5, or from about 4.5 to about 7.0, or from about 5.5 to about 7.0, or from about 4.0 to about 5.5, or from about 7.0 to about 9.5. In some embodiments, the organic acid inclusion is sufficient to provide a composition pH of about 4.0, about 4.5, about 5.0, about 5.5, about 6.0, about 6.5, about 7.0, about 7.5, about 8.0, about 8.5, or about 9.0. In some embodiments, the organic acid inclusion is sufficient to provide a composition pH of from about 4.5 to about 6.5, for example, from about 4.5, about 5.0, or about 5.5, to about 6.0, or about 6.5. In some embodiments, the organic acid is provided in a quantity sufficient to provide a pH of the composition of from about 5.5 to about 6.5, for example, from about 5.5, about 5.6, about 5.7, about 5.8, about 5.9, or about 6.0, to about 6.1, about 6.2, about 6.3, about 6.4, or about 6.5. In some embodiments, a mineral acid (e.g., hydrochloric acid, sulfuric acid, phosphoric acid, or the like) is added to adjust the pH of the composition to the desired value.

In some embodiments, the organic acid is added as the free acid, either neat (i.e., native solid or liquid form) or as a solution in, e.g., water, to the other oral composition components. In some embodiments, the alkali metal salt of the organic acid is added, either neat or as a solution in, e.g., water, to the other oral composition components.

In some embodiments, the organic acid is added as the free acid, either neat (i.e., native solid or liquid form) or as a solution in, e.g., water, to the other composition components. In some embodiments, the alkali metal salt of the organic acid is added, either neat or as a solution in, e.g., water, to the other composition components. In some embodiments, the organic acid and the basic amine (e.g., nicotine) are combined to form a salt, either before addition to the composition, or the salt is formed within and is present in the composition as such. In some embodiments, the organic acid and basic amine (e.g., nicotine) are present as individual components in the composition and form an ion pair upon contact with moisture (e.g., saliva in the mouth of the consumer).

In some embodiments, the composition comprises nicotine benzoate and sodium benzoate, wherein at least a portion of the nicotine and benzoate ions present are in an ion paired form. In some embodiments, the composition comprises nicotine benzoate, sodium benzoate, and an organic acid, an alkali metal salt of an organic acid, or a combination thereof, the organic acid having a logP value from about 1 to about 12, wherein the organic acid is a monoester of a dicarboxylic acid or is a carotenoid derivative having one or more carboxylic acids.

In some embodiments, the composition further comprises a solubility enhancer to increase the solubility of one or more of the organic acid or salt thereof. Suitable solubility enhancers include, but are not limited to, humectants as described herein, such as glycerol or propylene glycol.

In particular embodiments, the composition comprises nicotine benzoate and sodium benzoate. In some embodiments, the sodium benzoate is present in a molar ratio to the nicotine benzoate in a range from about 1 to about 20, such as about 2, about 5, or about 10. In some embodiments, the oral composition further comprises nicotine polacrilex. In some embodiments, the total amount of nicotine present in the composition is provided in equal quantities from nicotine benzoate and nicotine polacrilex. In some embodiments, the composition includes only nicotine polacrilex as the nicotine source, and the composition optionally comprises an organic acid component such as sodium benzoate.

### Binders

A binder (or combination of binders) may be employed in some embodiments, in amounts sufficient to provide the desired physical attributes and physical integrity to the composition. Typical binders can be organic or inorganic, or a combination thereof. Representative binders include povidone, sodium alginate, starch-based binders, pectin, carrageenan, pullulan, zein, and the like, &and combinations thereof.

The amount of binder utilized in the composition can vary, but is typically up to about 30 percent by weight, and some embodiments are characterized by a binder content of at least about 0.1 % by weight, such as about 1 to about 30% by weight or about 5 to about 10% by weight, based on the total weight of the composition.

In some embodiment, the composition comprises a cellulose derivative as a binder. In some embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In one embodiment, the cellulose derivative is one or more of methylcellulose, HPC, HPMC, hydroxyethyl cellulose, and CMC. In some embodiments, the cellulose derivative is HPC. In one embodiment, the cellulose derivative is a combination of HPC and HPMC. In some embodiments, the composition comprises from about 1 to about 5% by weight of HPC, for example, from about 1%, about 2%, or about 3%, to about 4%, or about 5% by weight of the composition.

In some embodiments, the composition comprises pectin as a binder. Pectins are natural polymers related to carbohydrates and which are acidic heteropolysaccharides (polysaccharides comprising multiple monosaccharide units). As opposed to carbohydrates, the pectin C-6 position contains a carboxylic acid (or corresponding methyl ester or carboxamide) group instead of a hydroxymethyl group. The principal subunit is known as galacturonic acid, which can be copolymerized with L-rhamnose. Other sugars are featured as side-chain substituents. Pectin acts as a thickening and gelling agent. Pectin isolated from sources such as apple pomace, citrus peels, sugarbeet waste from sugar manufacturing, sunflower heads discarded from seed harvesting, mango waste, and other commercially available pectins may be used. In combination with certain sugars, under acidic conditions (e.g., a pH of from about 2.5 to about 5), or in the presence of a gelation agent (calcium or other divalent alkaline earth elements), pectins may provide a gel or gum consistency to compositions as disclosed herein. In some embodiments, the binder comprises low methoxy pectin. Suitable low methoxy pectins include, for example, "GENU^{®} pectin type LM-104 AS", available from CP Kelco, Atlanta, GA, USA. In some embodiments, the binder comprises low methoxy pectin in combination with a gelation agent. In some embodiments, the gelation agent comprises calcium ions, such as, but not limited to, calcium diphosphate. In some embodiments, the binder comprises a high methoxy pectin in combination with an organic acid, described herein below. In some embodiments, the binder comprises a high methoxy pectin in combination with citric acid.

In some embodiments, the composition comprises an alginate as a binder, such as ammonium alginate, propylene glycol alginate, potassium alginate, or sodium alginate. Alginates, and particularly high viscosity alginates, may be employed in conjunction with controlled levels of free calcium ions.

In some embodiments, the composition includes a gum binder, for example, a natural gum. As used herein, a natural gum refers to polysaccharide materials of natural origin that have binding properties, and which are also useful as a thickening or gelling agents. Representative natural gums derived from plants, which are typically water soluble to some degree, include xanthan gum, guar gum, gum arabic, ghatti gum, gum tragacanth, karaya gum, locust bean gum, gellan gum, and combinations thereof. When present, natural gum binder materials are typically present in an amount of up to about 5% by weight, for example, from about 0.1, about 0.2, about 0.3, about 0.4, about 0.5, about 0.6, about 0.7, about 0.8, about 0.9, or about 1%, to about 2, about 3, about 4, or about 5% by weight, based on the total weight of the composition.

### Water

The water content of the composition or a product comprising the composition (e.g., a pouched product), prior to use by a consumer of the composition or product, may vary according to the desired properties. Typically, the composition, prior to insertion into the mouth of the user, includes less than about 60 percent by weight of water, and generally is from about 1 to about 60% by weight of water, for example, from about 5 to about 55, about 10 to about 50, about 20 to about 45, or about 25 to about 40 percent water by weight, based on the total weight of the product. In some embodiments, the product is at least about 5% water, for example, from about 5 to about 60% water by weight, based on the total weight of the composition. In some embodiments, the product is a pouched product comprising the composition, the pouched product having a water content from about 20 to about 60% by weight, based on the total weight of the product.

### Salts

In some embodiments, the composition may further comprise a salt (e.g., alkali metal salts), typically employed in an amount sufficient to provide desired sensory attributes to the composition. Non-limiting examples of suitable salts include sodium chloride, potassium chloride, ammonium chloride, flour salt, and the like. When present, a representative amount of salt is about 0.5 percent by weight or more, about 1.0 percent by weight or more, or at about 1.5 percent by weight or more, but will typically make up about 10 percent or less of the total weight of the composition, or about 7.5 percent or less or about 5 percent or less (e.g., about 0.5 to about 5 percent by weight).

### Humectants

In some embodiments, one or more humectants may be employed in the composition. Examples of humectants include, but are not limited to, glycerin, propylene glycol, and the like. Where included, the humectant is typically provided in an amount sufficient to provide desired moisture attributes to the mixture. Further, in some instances, the humectant may impart desirable flow characteristics to the mixture for depositing in a mold. When present, a humectant will typically make up about 5% or less of the weight of the composition (e.g., from about 0.5 to about 5%). When present, a representative amount of humectant is about 0.1% to about 1% by weight, or about 1% to about 5% by weight, based on the total weight of the composition.

### Buffering Agents

In some embodiments, the composition of the present disclosure can comprise pH adjusters or buffering agents. Examples of pH adjusters and buffering agents that can be used include, but are not limited to, metal hydroxides (e.g., alkali metal hydroxides such as sodium hydroxide and potassium hydroxide), and other alkali metal buffers such as metal carbonates (e.g., potassium carbonate or sodium carbonate), or metal bicarbonates such as sodium bicarbonate, and the like. Where present, the buffering agent is typically present in an amount less than about 5 percent based on the weight of the composition, for example, from about 0.1% to about 5%, such as, e.g., from about 0.2% to about 4%, from about 0.3% to about 3%, or from about 1% to about 2% by weight, based on the total weight of the composition. Non-limiting examples of suitable buffers include alkali metals acetates, glycinates, phosphates, glycerophosphates, citrates, carbonates, hydrogen carbonates, borates, or mixtures thereof.

### Colorants

A colorant may be employed in amounts sufficient to provide the desired physical attributes to the composition. Natural or synthetic colorants, such as natural or synthetic dyes, food-grade colorants and pharmaceutical-grade colorants may be used. Examples of colorants include various dyes and pigments, such as caramel coloring and titanium dioxide. Natural colorants such as curcumin, beet juice extract, spirulina may be used. Also, a variety of synthetic pigments may be used. In some embodiments, the colorant is a lake dye, such as a red or blue aluminum lake dye. The amount of colorant utilized in the composition can vary, but when present is typically up to about 3% by weight, such as from about 0.1%, about 0.5%, or about 1%, to about 3% by weight, based on the total weight of the composition.

### Tobacco Material

In some embodiments, the composition may include a tobacco material. Descriptions of various types of tobaccos, growing practices and harvesting practices are set forth in Tobacco Production, Chemistry and Technology, Davis et al. (Eds.) (1999), which is incorporated herein by reference. The tobacco material is typically used in a form that can be described as particulate (i.e., shredded, ground, granulated, or powder form). The manner by which the tobacco material is provided in a finely divided or powder type of form may vary. Preferably, plant parts or pieces are comminuted, ground or pulverized into a particulate form using equipment and techniques for grinding, milling, or the like.

In some embodiments, the type of tobacco material is selected such that it is initially visually lighter in color than other tobacco materials to some degree (e.g., whitened or bleached).

In various embodiments, the tobacco material can be treated to extract a soluble component of the tobacco material therefrom. "Tobacco extract" as used herein refers to the isolated components of a tobacco material that are extracted from solid tobacco pulp by a solvent that is brought into contact with the tobacco material in an extraction process. While a tobacco extract may comprise nicotine, reference to a tobacco extract herein is distinct and different from nicotine, which may also be obtained, e.g., by extraction from tobacco material. In various embodiments, a tobacco material can be treated to extract soluble components of the tobacco material therefrom.

When present, typical inclusion ranges for tobacco materials can vary depending on the nature and type of the tobacco material, and the intended effect on the final composition, with an example range of up to about 30% by weight, based on total weight of the composition (e.g., about 0.1 to about 15% by weight). In some embodiments, the composition comprises up to about 5% of tobacco, for example, from about 0.1 to about 1%, or from about 1% to about 5% by weight of tobacco, based on the total weight of the composition. In some embodiments, the composition comprises a traditional tobacco or a white tobacco. In some embodiments, the tobacco is a white tobacco. In some embodiments, the compositions of the disclosure can be characterized as completely free or substantially free of tobacco material (other than purified nicotine as an active ingredient). For example, some embodiments can be characterized as having less than 1% by weight, or less than 0.5% by weight, or less than 0.1% by weight of tobacco material, or 0% by weight of tobacco material.

### Other Additives

Other additives can be included in the disclosed compositions. For example, the compositions can be processed, blended, formulated, combined and/or mixed with other materials or ingredients. The additives can be artificial or can be obtained or derived from herbal or biological sources. Examples of types of additives include gelling agents (e.g., fish gelatin), emulsifiers, oral care additives (e.g., thyme oil, eucalyptus oil, and zinc), preservatives (e.g., potassium sorbate and the like), antioxidants, disintegration aids, or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., U.S. Pat. App. Pub. No. 2010/0291245 to Gao et al., and U.S. Pat. App. Pub. No. 2007/0062549 to Holton, Jr. et al., each of which is incorporated herein by reference. These and other exemplary types of additives may include those described in, for example, previously incorporated by reference herein. Typical inclusion ranges for such additional additives can vary depending on the nature and function of the additive and the intended effect on the final composition, with an example range of up to about 10% by weight, based on total weight of the composition (e.g., about 0.1 to about 5% by weight).

The aforementioned additives can be employed together (e.g., as additive formulations) or separately (e.g., individual additive components can be added at different stages involved in the preparation of the final composition). Furthermore, the aforementioned types of additives may be encapsulated as provided in the final product or composition. Exemplary encapsulated additives are described, for example, in WO 2010/132444 to Atchley, incorporated by reference herein.

### Configured for Oral Use

Provided herein are compositions configured for oral use. The term "configured for oral use" as used herein means that the composition or product including such composition is provided in a form such that during use, saliva in the mouth of the user causes one or more of the releasable components of the composition (e.g., flavoring agents and/or active ingredient) to pass into the mouth of the user. In some embodiments, the composition or product is adapted to deliver components to a user through mucous membranes in the user's mouth and, in some instances, said component is an active ingredient (including, but not limited to, for example, nicotine) that can be absorbed through the mucous membranes in the mouth when the composition or product is used. In some embodiments, the component is a flavoring agent.

Products configured for oral use as described herein may take various forms, including gels, pastilles, gums, lozenges, powders, and pouches. Certain products of the disclosure are in the form of solids. Certain products can exhibit, for example, one or more of the following characteristics: crispy, granular, chewy, syrupy, pasty, fluffy, smooth, and/or creamy. In some embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

The compositions as disclosed herein can be formed into a variety of shapes, including pills, tablets, spheres, strips, films, sheets, coins, cubes, beads, ovoids, obloids, cylinders, bean-shaped, sticks, or rods. Cross-sectional shapes of the composition can vary, and example cross-sectional shapes include circles, squares, ovals, rectangles, and the like.

The compositions of the present disclosure may be at least partially dissolvable. As used herein, the terms "dissolve," "dissolving," and "dissolvable" refer to compositions having aqueous-soluble components that interact with moisture in the oral cavity and enter into solution, thereby causing gradual consumption of the composition. According to one aspect, the dissolvable composition is capable of lasting in the user's mouth for a given period of time until it completely dissolves. Dissolution rates can vary over a wide range, from about 1 minute or less to about 60 minutes. For example, fast release compositions typically dissolve and/or release the active substance in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the composition.

In some embodiments, the composition is in the form of a compressed or molded pellet. Example pellet weights range from about 250 mg to about 1500 mg, such as about 250 mg to about 700 mg, or from about 700 mg to about 1500 mg. The pellet can have any of a variety of shapes, including traditional pill or tablet shapes.

In some embodiments, the composition may be in the form of a dissolvable and lightly chewable pastille. As used herein, the term "pastille" refers to a dissolvable oral product made by solidifying a liquid or gel composition, such as a composition that includes a gelling or binding agent, so that the final product is a hardened solid gel. A pastille product may alternatively be referred to as a soft lozenge. In some embodiments, the pastille products of the disclosure are characterized by sufficient cohesiveness to withstand light chewing action in the oral cavity without rapidly disintegrating. The pastille products of the disclosure typically do not exhibit a highly deformable chewing quality as found in conventional chewing gum. See, for example, the smokeless tobacco pastilles, pastille formulations, pastille configurations, pastille characteristics and techniques for formulating or manufacturing pastilles set forth in US Pat. Nos. 9,204,667 to Cantrell et al.; 9,775,376 to Cantrell et al.; 10,357,054 to Marshall et al.; which are incorporated herein by reference.

In some embodiments, the products disclosed herein may be in the form of a dissolvable lozenge product configured for oral use. Lozenge products are generally described as "hard" and are distinguished in this manner from soft lozenges (i.e., pastilles). Example lozenge-type products of the disclosure have the form of a lozenge, tablet, microtab, or other tablet-type product. See, for example, the types of nicotine-containing lozenges, lozenge formulations, lozenge formats and configurations, lozenge characteristics and techniques for formulating or manufacturing lozenges set forth in US Pat. Nos. 4,967,773 to Shaw; 5,110,605 to Acharya; 5,733,574 to Dam; 6,280,761 to Santus; 6,676,959 to Andersson et al.; 6,248,760 to Wilhelmsen; and 7,374,779; US Pat. Pub. Nos. 2001/0016593 to Wilhelmsen; 2004/0101543 to Liu et al.; 2006/0120974 to Mcneight; 2008/0020050 to Chau et al.; 2009/0081291 to Gin et al.; and 2010/0004294 to Axelsson et al.; which are incorporated herein by reference.

In some embodiments, the composition can be chewable, meaning the composition has a mild resilience or "bounce" upon chewing, and possesses a desirable degree of malleability. A composition in chewable form may be entirely dissolving or may be in the form of a non-dissolving gum in which only certain components (e.g., active ingredients, flavor, sweetener) dissolve, leaving behind a non-dissolving matrix.

In some embodiments, the composition can be meltable as discussed, for example, in US Patent App. Pub. No. 2012/0037175 to Cantrell et al., incorporated by reference herein in its entirety. As used herein, "melt," "melting," and "meltable" refer to the ability of the composition to change from a solid state to a liquid state. That is, melting occurs when a substance (e.g., a composition as disclosed herein) changes from solid to liquid, usually by the application of heat. The application of heat in regard to a composition as disclosed herein is provided by the internal temperature of a user's mouth. Thus, the term "meltable" refers to a composition that is capable of liquefying in the mouth of the user as the composition changes phase from solid to liquid, and is intended to distinguish compositions that merely disintegrate in the oral cavity through loss of cohesiveness within the composition that merely dissolve in the oral cavity as aqueous-soluble components of the composition interact with moisture.

In some embodiments, the composition of the present disclosure is disposed within a moisture-permeable container (e.g., a water-permeable pouch) to form a pouched oral product. Such pouched products are typically used by placing one pouch containing the composition in the mouth of a human subject/user. Generally, the pouch is placed somewhere in the oral cavity of the user, for example under the lips, in the same way as moist snuff products are generally used. The pouch preferably is not chewed or swallowed. Exposure to saliva then causes some of the components of the composition therein (e.g., flavoring agents and/or nicotine) to pass through e.g., the water-permeable pouch and provide the user with flavor and satisfaction, and the user is not required to spit out any portion of the composition. After about 10 minutes to about 60 minutes, typically about 15 minutes to about 45 minutes of use/enjoyment, substantial amounts of the composition have been ingested by the human subject, and the pouch may be removed from the mouth of the human subject for disposal.

Accordingly, in some embodiments, the composition as disclosed herein and any other components noted above are combined within a moisture-permeable packet or pouch that acts as a container for use of the composition to provide a pouched product configured for oral use. Some embodiments of the disclosure will be described with reference to the Figure, which provides an illustration of an example pouched product. Referring to the Figure, there is shown an embodiment of a pouched product **100.** The pouched product **100** includes a moisture-permeable container in the form of a pouch **102** housing a material **104** comprising the oral composition described herein.

An example pouch **102** can be constructed of a fleece material, which is a fibrous material that can be formed from various types of fibers (e.g., conventional cellulosic fibers (e.g., such as viscose fibers, regenerated cellulose fibers, cellulose fibers, and wood pulps), cotton fibers, wool fibers, other natural fibers, polymer/synthetic-type fibers, and combinations thereof) capable of being formed into a fabric. For example, fleece materials may be provided in the form of a woven or nonwoven fabric. Suitable types of fleece materials, for example, are described in U.S. Patent No. 8,931,493 to Sebastian et al.; US Patent App. Pub. No. 2016/0000140 to Sebastian et al.; and US Patent App. Pub. No. 2016/0073689 to Sebastian et al., which are all incorporated herein by reference.

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and composition each may undergo complete dispersion within the mouth of the user during normal conditions of use, and hence the pouch and composition both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). If desired, flavoring ingredients, disintegration aids, and other desired components, may be incorporated within, or applied to, the pouch material.

The amount of material contained within each product unit, for example, a pouch, may vary. In some embodiments, the dry weight of the composition within each pouch is at least about 50 mg, for example, from about 50 mg to about 1 gram, from about 100 to 800 about mg, or from about 200 to about 700 mg. In some smaller embodiments, the dry weight of the composition within each pouch may be from about 100 to about 300 mg. For a larger embodiment, the dry weight of the material within each pouch may be from about 300 mg to about 700 mg. If desired, other components can be contained within each pouch. For example, at least one flavored strip, piece or sheet of flavored water dispersible or water-soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference.

### Preparation of the Composition

The manner by which the various components of the composition are combined and/or mixed may vary. As such, the overall composition with e.g., powdered composition components may be relatively uniform in nature (e.g., homogenous). The components noted above, which may be in liquid or dry solid form, can be admixed in a pretreatment step prior to mixture with any remaining components of the composition, or simply mixed together with all other liquid or dry ingredients. The compositions of the disclosure are prepared, for example, by dry-blending dry ingredients, such as filler, sweeteners, salts, and the like. In some embodiments, water can be added to the dry blend at this stage. Additionally, it is optional to add, such as by spraying or mixing, active ingredients and/or flavoring agents to the dry blend, followed by mixing.

The various components of the composition may be contacted, combined, or mixed together using any mixing technique or equipment known in the art. Any mixing method that brings the composition ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in U.S. Pat. Nos. 4,148,325 to Solomon et al.; 6,510,855 to Korte et al.; and 6,834,654 to Williams, each of which is incorporated herein by reference. Manners and methods for formulating mixtures will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

In some embodiments, the composition is enclosed in a pouch to form a pouched product as described herein above. In some embodiments, the composition is in a free-flowing, particulate form. Accordingly, in some embodiments, the method further comprises enclosing the product in a pouch to form the pouched product.

Many modifications and other embodiments of the disclosure will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

### EXAMPLES

Aspects of the present disclosure are more fully illustrated by the following examples, which are set forth to illustrate certain aspects of the present disclosure and are not to be construed as limiting thereof.

### Example 1. Preparation of a pouched oral product

A pouched oral product comprising a composition according to present disclosure comprising nicotine as the active ingredient was prepared. The composition included nicotine, microcrystalline cellulose (MCC), sweeteners (saccharin, xylitol, Acesulfame K), water, and additional components as disclosed herein (salt, humectant, buffer, flavoring agent). The composition had the formulation provided in **Table 2.**

To prepare the composition, a dry blend of MCC, alginate, salt, sodium bicarbonate, and sweeteners was mixed in a blender. To this dry mix was added a mixture of water, nicotine, and propylene glycol with mixing. To the wet mix was added flavorant and additional water to provide a final composition moisture content of approximately 14% by weight. Portions of the composition were placed in pouches (420 mg total pouch weight) and water was added to bring the total moisture content to 30-40% by weight.

**Table 2. Composition ingredients**

| **Ingredient** | **% by weight of composition** |
|---|---|
| microcrystalline cellulose | 55-65 |
| water | 30-40 |
| nicotine | 0.5-1.5 |
| xylitol | 1-3 |
| acesulfame K | 0.1-0.5 |
| saccharin | 0.05-0.3 |
| sodium chloride | 1-3 |
| propylene glycol | 0.3-1.2 |
| sodium bicarbonate | 0.1-0.5 |
| flavoring agent | 0.3-1.2 |

### Example 2. Preparation of pouched oral product

A pouched oral product comprising a composition according to present disclosure comprising nicotine as the active ingredient was prepared. The composition included nicotine, microcrystalline cellulose (MCC), alginate binder, sweeteners (saccharin, xylitol, Acesulfame K), water, and additional components as disclosed herein (salt, humectant, buffer, flavoring agent). The composition had the formulation provided in **Table 3.**

To prepare the compositions, a dry blend of MCC, alginate, salt, sodium bicarbonate, and sweeteners is mixed in a blender. To this dry mix is added a mixture of water, nicotine, and propylene glycol with mixing. To the wet mix is added flavorant and additional water to provide a final composition moisture content of approximately 14% by weight. Portions of the composition are placed in pouches (500 mg total pouch weight) and water is added to bring the total moisture content to 35-45% by weight.

**Table 3. Composition ingredients**

| **Ingredient** | **% by weight of composition** |
|---|---|
| microcrystalline cellulose | 40-55 |
| water | 35-45 |
| nicotine | 0.5-1.5 |
| xylitol | 1-3 |
| acesulfame K | 0.1-0.5 |
| saccharin | 0.05-0.3 |
| sodium chloride | 1-3 |
| sodium alginate | 0.5-1.0 |
| propylene glycol | 0.5-1.5 |
| sodium bicarbonate | 0.1-0.4 |
| flavoring agent | 1-3 |

## Claims

1. A pouched product configured for oral use, comprising a composition enclosed within a water-permeable pouch, the composition comprising: a particulate filler component; one or more sweeteners; and a releasable component selected from the group consisting of flavoring agents, active ingredients, and combinations thereof, wherein the composition is substantially free of sucralose; optionally, the composition further comprising one or more salts, one or more organic acids, one or more binding agents, one or more buffering agents, one or more humectants, or combinations thereof.

2. The pouched product of claim 1, wherein the one or more sweeteners comprises at least one artificial, non-nutritive sweetener.
%

3. The pouched product of claim 2, wherein the at least one artificial, non-nutritive sweetener is selected from the group consisting of saccharin, aspartame, neotame, Advantame, cyclamate, acesulfame K, and combinations thereof.

4. The pouched product of any one of claims 1-3, wherein the one or more sweeteners comprise a sugar alcohol, steviol or an isosteviol glycoside, thaumatin, D-psicose, rubusoside, rebaudioside B, rebaudioside C, trilobatin, phyllodulcin, brazzein, mogrosides, or combinations thereof.

5. The pouched product of any one of claims 1-3, wherein the one or more sweeteners comprises a combination of xylitol, acesulfame K, and saccharin.

6. The pouched product of claim 5, comprising:
about 1 to about 5% by weight of xylitol;
about 0.1 to about 0.75% by weight of acesulfame K; and
about 0.05 to about 0.5% by weight of saccharin, based on the total weight of the composition.

7. The pouched product of any one of claims 1-6, wherein the particulate filler component comprises a cellulose material, a starch, or both.

8. The pouched product of claim 7, wherein the particulate filler component comprises microcrystalline cellulose.

9. The pouched product of any one of claims 1-8, having a water content from about 20 to about 60% by weight, based on the total weight of the pouched product.

10. The pouched product of any one of claims 1-9, wherein the releasable component comprises a flavoring agent.

11. The pouched product of any one of claims 1-9, wherein the releasable component comprises one or more active ingredients selected from the group consisting of nutraceuticals, botanicals, stimulants, amino acids, vitamins, minerals, cannabinoids, cannabimimetics, terpenes, and combinations thereof.

12. The pouched product of any one of claims 1-9, wherein the releasable component comprises a nicotine component.

13. The pouched product of any one of claims 1-12, further comprising a sweetness enhancer and/or a bitter modulator, optionally, wherein the sweetness enhancer is a positive allosteric modulator of the T1R2/T1R3 taste receptor, and optionally when the sweetness enhancer is FEMA 4774.

14. The pouched product of claim 13, wherein the bitter modulator is selected from the group consisting of capsaicin, gamma-amino butyric acid (GABA), adenosine monophosphate (AMP), and combinations thereof.

15. The pouched product of any one of claims 1-14, comprising:
about 30 to about 75% by weight of microcrystalline cellulose,
about 20 to about 60% by weight of water;
about 0.1 to about 10% by weight of a nicotine component, calculated as the free base;
about 1 to about 5% by weight of xylitol;
about 0.1 to about 0.75% by weight of acesulfame K; and
about 0.05 to about 0.5% by weight of saccharin, based on the total weight of the composition.
